# FASCICULE DE BREVET EUROPEEN

(11) **EP 0 432 001 B1**
(45) Date de publication et mention de la délivrance du brevet: **07.02.1996**
(21) Numéro de dépôt: 90403268.7
(22) Date de dépôt: 20.11.1990
(51) Int. Cl.: A61C 13/30

(54) **Tenon d'ancrage dentaire physiologique en matériau composite et son procédé de fabrication**
Anker für die Dental-Prothetik aus vielfältigem Material und sein Herstellungsverfahren
Root pin for a dental prosthesis made from composite material and its procedure of manufacture

(30) Priorité: 20.11.1989 FR 8915194
(43) Date de publication de la demande: 12.06.1991
(73) Titulaire: Reynaud, Marc, F-38240 Meylan (FR); Reynaud, Pierre-Luc, F-38000 Grenoble (FR); Duret, François, F-38690 Le-Grand-Lemps (FR); Duret, Bernard, F-38470 Vinay (FR)
(72) Inventeur: Reynaud, Marc, F-38240 Meylan (FR); Reynaud, Pierre-Luc, F-38000 Grenoble (FR); Duret, François, F-38690 Le-Grand-Lemps (FR); Duret, Bernard, F-38470 Vinay (FR)
(74) Mandataire: Bruder, Michel

(56) Documents cités:
- DE-A- 3 825 601
- FR-A- 2 173 350
- FR-A- 2 588 181
- FR-A- 2 626 167

## Description

La présente invention concerne un ensemble de reconstitution coronoradiculaire selon le préambule de la revendication 1, ainsi qu'un procédé de fabrication de ce tenon selon la revendication 8.

Un ensemble de reconstitution coronoradiculaire comme défini dans le préambule de la revendication 1 est exposé dans le brevet FR-A-2626167.

Les tenons d'ancrage dentaires, qui sont actuellement utilisés couramment en odontologie, sont généralement réalisés en alliages métalliques. Toutefois ces tenons ne se sont pas révélés totalement satisfaisants car même s'ils sont réalisés en alliage précieux, ce qui est souvent le cas pour des raisons réglementaires, ils peuvent donner lieu à des phénomènes de corrosion et d'oxydation d'origine chimique ou électrochimique.

Pour remédier à cet inconvénient on a déjà envisagé, comme cela est décrit dans le brevet FR-A-2 588 181, de réaliser une prothèse endobuccale en matériau composite constituée de fibres de haute résistance mécanique, incluses dans une résine constituant la matrice du matériau. On connaît également, ainsi qu'il est décrit dans le brevet FR-A-2626167, un tenon comportant un fil ou filament central autour duquel est moulée une résine synthétique qui contient de préférence des fibres. On peut donner aux tenons fabriqués de cette manière des caractéristiques physiques et mécaniques intéressantes mais les tenons ainsi obtenus ne sont pas totalement satisfaisants car leurs propriétés mécaniques sont assez éloignées des caractéristiques naturelles de la dentine. La disparité des propriétés mécaniques entre celles du tenon et celles de la dentine est une source de perturbation constante, avec une concentration des contraintes en certains points, et il peut en résulter la destruction de l'ensemble de reconstruction, si les contraintes appliquées à cet ensemble, au cours de la mastication, arrivent à dissocier les éléments constitutifs.

La présente invention vise à remédier à cet inconvénient en procurant un ensemble de reconstitution permettant d'amortir les contraintes et de les répartir au maximum dans les zones soumises aux efforts.

A cet effet cet ensemble de reconstitution coronoradiculaire comportant un tenon d'ancrage dentaire physiologique en matériau composite, et un matériau de reconstitution coronaire destiné à être mis en place entre le tenon et les parois de dentine saine, le tenon comportant des fibres de haute résistance noyées dans une résine constituant la matrice, les fibres étant sensiblement parallèles entre elles et continues d'une extrémité à l'autre, est caractérisé en ce que les fibres sont allongées dans le sens axial du tenon et équitendues, de manière que le tenon ainsi obtenu présente des caractéristiques mécaniques voisines de celles de la dentine en ce qui concerne le module d'élasticité transversal et supérieures à celles de la dentine en ce qui concerne la résistance à la compression ou traction dans le sens longitudinal et la résistance au cisaillement.

Un tenon d'ancrage suivant l'invention présente des caractéristiques mécaniques anisotropes, c'est-à-dire qui dépendent fortement de la direction d'application d'un effort par rapport à la direction longitudinale des fibres, c est-à-dire du tenon. Ainsi un tel tenon réalisé à partir de fibres en carbone haute performance noyées dans une matrice de résine époxy, a un module d'élasticité transversal, qui a une valeur de 8,5 GPa pour un angle d'application de l'effort de traction, par rapport à la direction transversale, de 0° et une valeur de 34 GPa pour un angle de 20°, ce qui donne une moyenne de 21 GPa dans la plage angulaire de 0° à 20° qui correspond à l'orientation moyenne des sollicitations s'exerçant sur une dent pendant la mastication. Cette valeur est voisine du module d'élasticité transversal de la dentine qui est de 18 GPa. En ce qui concerne la résistance à la compression elle peut aller jusqu'à 500 MPa c'est-à-dire 200 MPa de plus que la résistance moyenne de la dentine. De telles caractéristiques sont obtenues avec des matériaux composites dont 100% des fibres sont continues d'un bout à l'autre du tenon et équitendues, ces fibres représentant de 40 à 80% du volume total du tenon c'est-à-dire du volume des fibres elles-mêmes et de la résine constituant la matrice. Les fibres sont de préférence des fibres de carbone haute performance et les résines sont de préférence des résines époxy ou polyesters. En général de tels matériaux composites ont une masse volumique de l'ordre de 1,50 à 1,60 g/cm3. Les caractéristiques ci-dessus indiquées sont obtenues après usinage, ce qui les différencie des matériaux de l'art antérieur qui perdent une grande partie de leurs caractéristiques lors de l'usinage des joncs obtenus, même lorsqu'ils sont constitués en matériau composite formé de fibres noyées dans une résine dès lors qu'intervient un usinage et que la nature du matériau n'est pas conçue de façon appropriée pour présenter l'anisotropie nécessaire et orientée correctement.

Pour mieux faire comprendre les caractéristiques techniques et les avantages de la présente invention on va en décrire un exemple de réalisation, à titre d'exemple non limitatif, en référence aux dessins annexés sur lesquels :
La figure 1 est un vue en coupe verticale et longitudinale d'un ensemble de reconstitution coronoradiculaire suivant l'invention.
La figure 2 est une reproduction schématique d'une micrographie d'une partie du tenon de l'ensemble suivant l'invention, obtenue au moyen d'un microscope électronique à balayage, avec un grandissement de 500.
La figure 3 est une vue en coupe horizontale et transversale partielle, à plus grande échelle, faite suivant la ligne III-III de la figure 1.
La figure 4 est une vue en coupe transversale, à plus grande échelle encore, d'une partie de la racine d'une dent et du tenon d'ancrage.

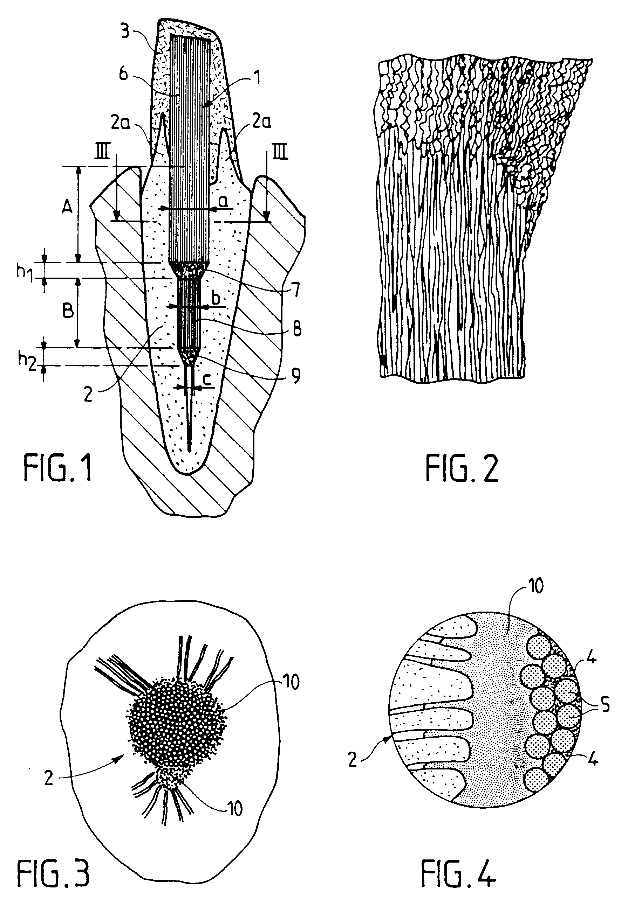

Sur la figure 1 est représenté un ensemble de reconstitution coronoradiculaire suivant l'invention lequel est constitué d'un tenon d'ancrage physiologique 1, en matériau composite, engagé et immobilisé dans un logement foré dans la lumière canalaire de la racine d'une dent 2, et d'un bloc de matériau de reconstitution coronaire 3 formé sur la partie supérieure du tenon 1, qui fait saillie au-dessus de la racine 2.

Pour obtenir le tenon d'ancrage 1 on réalise tout d'abord un jonc en matériau composite par pultrusion (ou tout autre procédé équivalent), c'est-à-dire par extrusion d'une résine époxy 4 autour d'une mèche de fibres continues 5 de carbone haute performance, de 8 micromètres de diamètre, ces fibres 5 représentant de 40 à 80% et de préférence 60% du volume total à la sortie de l'extrudeuse, c'est-à-dire du volume comprenant les fibres et la résine après sa solidification. Les fibres 5 sont équitendues et à disposition longitudinale unidirectionnelle dans l'axe du tenon 1, elles ne présentent ni boucles, ni guipures, et en outre elles sont légèrement précontraintes en traction.

Dans une forme d'exécution particulièrement avantageuse de l'invention les fibres équitendues occupent 64% du volume total ou du poids total car la masse volumique de ces fibres est sensiblement égale à celle de la résine époxy 4. Ce taux de fibres très élevé donne au tenon 1 des valeurs en cisaillement exceptionnellement élevées, (résistance du cisaillement 170MPa) qui évitent toute fracture du tenon, dans des conditions physiologiques normales. Sa résistance en compression qui est de 440 MPa, le situe légèrement au-dessus des structures dentaires associées. Par ailleurs l'anisotropie de comportement mécanique du matériau du tenon 1 a permis de reproduire avec beaucoup de similitude le décrément du module d'élasticité de la dentine coronaire à la dentine radiculaire, soit de 80 GPa à 18 GPa, lors des interférences occlusales. Du fait de l'anisotropie du matériau constitutif du tenon, le module d'élasticité en traction-compression est variable suivant l'angle d'application de l'effort par rapport à l'axe des fibres 5. Cette propriété est fondamentale car c'est elle qui module les effets des contraintes et permet d'augmenter sans danger la surface de l'interface tenon/dentine. Si la contrainte est perpendiculaire aux fibres, le module d'élasticité est de 8 GPa. Si la contrainte fait un angle de 20° par rapport au plan transversal, le module d'élasticité est de 34 GPa. C'est le cas le plus fréquent lors des contraintes élevées de la mastication celles qui provoquent fissures et fractures à l'instant où l'on broie les aliments avant le retour en position centrée. A ce moment, le module d'élasticité a une valeur moyenne de 21 GPa qui est très proche du module d'élasticité de la dentine radiculaire (18 GPa).

Le jonc continu en matériau composite tel que défini ci-dessus, qui a un diamètre égal ou supérieur au plus grand diamètre des tenons devant être obtenus, est ensuite usiné, par exemple mécaniquement par décolletage, pour obtenir un tenon 1 ayant la forme représentée sur la figure 1. Ce tenon d'ancrage est constitué d'une pièce de révolution formée de deux parties cylindroconiques coaxiales et dans le prolongement l'une de l'autre, à savoir une partie cylindroconique supérieure de grand diamètre et une partie cylindroconique inférieure de plus petit diamètre. La partie cylindroconique supérieure comprend une tige cylindrique 6, de diamètre relativement grand a,prolongée, à son extrémité inférieure, par une partie tronconique 7, convergeant vers le bas, à petite base de diamètre b. A cette petite base fait suite une tige cylindrique 8 de diamètre b laquelle se termine par une partie tronconique 9, convergeant vers le bas et dont la petite base a un diamètre c. La tige cylindrique supérieure 6, de grand diamètre a, permet d'assurer une rétention cylindrique à l'intérieur de la racine 2 sur une distance A de l'ordre de 3 mm. Le tenon d'ancrage suivant l'invention permet d'assurer également une seconde rétention cylindrique sur la longueur B correspondant à la longueur de la tige cylindrique 8 de petit diamètre b. Les parties tronconiques 5 et 7 qui ont des hauteurs respectives h1 et h2, constituent des étages de stabilisation du tenon 1 dans la racine de la dent 2.

Dans le tenon d'ancrage tel qu'il vient d'être décrit les fibres équitendues 8 s'étendent dans le sens longitudinal et elles sont continues d'une face frontale du tenon à l'autre, en dehors bien entendu des fibres qui sont coupées dans les zones tronconiques 5 et 7.

Le tableau ci-dessous donne les valeurs des dimensions, en mm, de divers tenons qui ont donné toute satisfaction au cours des essais qui ont été effectuée.

| | | | |
|---|---|---|---|
| Diamètre a | 1,4 | 1,8 | 2,1 |
| Diamètre b | 1 | 1,2 | 1,4 |
| Diamètre c | 0,65 | 0,85 | 0,85 |
| Hauteur h1 | 1 | 1 | 1 |
| Hauteur h2 | 1 | 1 | 1 |
| Longueur B | 2,5 | 3 | 3,5 |

Comme il apparaît plus clairement sur les figures 2 et 4, le tenon obtenu après décolletage présente une surface irrégulière sur laquelle sont régulièrement répartis des accidents qui n'excédent jamais 10 à 20 micromètres mais dont la présence est constante. Ils sont dus, au niveau des étages de stabilisation tronconiques 5,7 du tenon, à la section oblique (30°) des fibres de carbone 5 par suite de l'usinage, et, sur les parties cylindriques, par des sillons entre les fibres 5 restées très solidement fixées à la matrice de résine époxy 4, ce qui crée ainsi des anfractuosités qui répliquent parfaitement à la surface dentinaire préparée.

Les accidents de la surface du tenon 1 donnent à celui-ci une rugosité superficielle améliorant l'adhérence d'une colle 10 (figure 3 et 4) dont on enduit les surfaces dentinaires et le tenon 1 avant la pose de celui-ci dans le logement de forage préalablement ouvert dans la lumière canalaire.

L'ensemble de reconstitution coronoradiculaire comprend également, comme il est représenté sur la figure 1, un élément de conjonction 3 entre le tenon 1 et les parois de dentine saine restantes 2a pour terminer la reconstruction de la perte de substance et pour s'adapter à la morphologie individuelle. Le matériau de reconstitution coronaire 3 utilisé à cet effet est une résine polyuréthane-acrylique autopolymérisante chargée de fibres de verre courtes ensimées (ayant subit un traitement de surface spécifique pour les lier solidement à la matrice polyuréthane acrylique), de 200 micromètres de longueur et de 9 micromètres de diamètre (60% en poids et 70 à 80% en volume). Ces fibres courtes sont disposées de façon aléatoire dans la masse du matériau 3. Les propriétés mécaniques du matériau de reconstitution 3 ont été établies pour permettre l'amortissement des contraintes et des liaisons durables entre le tenon 1 et les parois de dentine saine 2a. Son module d'élasticité très bas (3,7 ±0,3 GPa) lui confère des propriétés d'élasticité qui en font un amortisseur primaire de contraintes lors des premiers chocs dento-dentaires. Cette propriété a été très clairement mise en évidence lors de tests mécaniques en compression et en flexion où l'on a vu les éprouvettes dépasser la limite à la rupture, sans modification apparente du volume extérieur, dans 30% des cas. Sa résistance à la compression est proche de celle de la dentine coronaire (220 ± 20 MPa).

Son coefficient d'absorption d'eau est de 0,3% et son temps de prise est situé entre 3 et 4 minutes. Ce matériau est en outre chimiquement compatible avec le matériau composite carbone/époxy, ce qui lui permet d'établir des interfaces solides consolidées par les microrugosités de surface du tenon qui ajoutent les effets d'un microclavetage à la liaison chimique.

D'après la description qui précède on voit que les propriétés mécaniques des composants de l'ensemble de reconstitution coronoradiculaire suivant l'invention sont les plus voisines possibles les unes des autres, notamment en ce qui concerne le module d'élasticité transversal qui est très proche de celui de la dentine. De cette homogénéité du comportement découle une bonne cohésion entre les composants ainsi qu'une moindre fatigue des interfaces. Le matériau de reconstitution 3 joue le rôle d'amortisseur primaire des contraintes et cet amortissement est complété dans le tenon 1 du fait que son module d'élasticité, pour les contraintes s'exerçant dans la zone angulaire allant de 0° à 20° par rapport à un plan transversal, a une valeur moyenne sensiblement égale à celle de la dentine.

## Revendications

1. Ensemble de reconstitution coronoradiculaire comportant un tenon d'ancrage dentaire physiologique (1) en matériau composite, et un matériau de reconstitution coronaire (3) destiné à être mis en place entre le tenon (1) et les parois de dentine saine (2a), le tenon (1) comportant des fibres de haute résistance (5) noyées dans une résine (4) constituant la matrice, les fibres (5) étant sensiblement parallèles entre elles et continues d'une extrémité à l'autre, caractérisé en ce que les fibres (5) sont allongées dans le sens axial du tenon (1) et équitendues de manière que le tenon ainsi obtenu présente des caractéristiques mécaniques voisines de celles de la dentine en ce qui concerne le module d'élasticité transversal et supérieures à celles de la dentine en ce qui concerne la résistance à la compression ou traction dans le sens longitudinal et la résistance au cisaillement.

2. Ensemble de reconstitution coronoradiculaire suivant la revendication 1 caractérisé en ce qu'il est constitué de fibres de carbone (5) noyées dans une matrice en résine époxy (4).

3. Ensemble de reconstitution coronoradiculaire suivant l'une quelconque des revendications précédentes caractérisé en ce que les fibres (5) sont soumises à une même précontrainte de traction.

4. Ensemble de reconstitution coronoradiculaire suivant l'une quelconque des revendications précédentes caractérisé en ce que les fibres (5) représentent de 40 à 80% du volume total du tenon (1).

5. Ensemble de reconstitution coronoradiculaire suivant l'une quelconque des revendications précédentes dans lequel le tenon est constitué d'une pièce de révolution formée de deux parties cylindroconiques coaxiales et dans le prolongement l'une de l'autre, à savoir une partie cylindroconique supérieure de grand diamètre et une partie cylindroconique inférieure de plus petit diamètre, caractérisé en ce que le tenon (1) obtenu après décolletage présente une surface irrégulière sur laquelle sont régulièrement répartis des accidents qui n'excédent jamais 10 à 20 micromètres mais dont la présence est constante, ces accidents étant dus, au niveau des parties tronconiques (5,7) du tenon, à la section oblique des fibres (5) par suite de l'usinage, et, sur les parties cylindriques, par des sillons entre les fibres (5) restées très solidement fixées à la matrice de résine (4).

6. Ensemble de reconstitution coronoradiculaire suivant l'une quelconque des revendications précédentes caractérisé en ce que le matériau de reconstitution coronaire (3) est une résine polyuréthane-acrylique autopolymérisante chargée de fibres de verre courtes ensimées.

7. Ensemble de reconstitution coronoradiculaire suivant la revendication 6 caractérisé en ce que les fibres de verre ont environ une longueur de 200 micromètres et un diamètre de 9 micromètres, elles sont présentes à raison de 70 à 80% en volume et elles sont réparties d'une façon aléatoire dans la masse du matériau de reconstruction coronaire (3).

8. Procédé de fabrication d'un tenon d'ancrage dentaire physiologique en matériau composite pour un ensemble de reconstitution coronoradiculaire suivant l'une quelconque des revendications 1 à 7 caractérisé en ce qu'on extrude une résine (4) autour d'une mèche de fibres continues (5) équitendues, éventuellement précontraintes en traction, et, après durcissement de la résine, on découpe le jonc de matériau composite et on usine le tenon mécaniquement.

## Claims

1. Coronoradicular reconstitution assembly, comprising a physiological dental root pin (1) made of a composite material, and a coronary reconstitution material (3) intended to be placed between the pin (1) and the healthy dentine walls (2a), the pin (1) comprising high-resistance fibers (5) embedded in a resin (4) constituting the matrix, the fibers (5) being substantially parallel to one another and continuous from one end to the other, characterized in that the fibers (5) are elongated axially with respect to the pin (1) and in equal tension so that the pin thus obtained presents mechanical characteristics close to those of the dentine concerning the modulus of transverse elasticity and greater than those of the dentine concerning the lengthwise compressive or tensile strength and shear strength.

2. Coronoradicular reconstitution assembly according to Claim 1, characterized in that it is constituted by carbon fibers (5) embedded in an epoxy resin matrix (4).

3. Coronoradicular reconstitution assembly according to either of the preceding Claims, characterized in that the fibers (5) are subjected to the same traction prestressing.

4. Coronoradicular reconstitution assembly according to any one of the preceding Claims, characterized in that the fibers (5) represent from 40 to 80% of the total volume of the pin (1).

5. Coronoradicular reconstitution assembly according to any one of the preceding Claims, in which the pin is constituted by a piece of revolution formed by two coaxial cylindro-conical parts in line with one another, namely an upper cylindro-conical part of large diameter and a lower cylindro-conical part of smaller diameter, characterized in that the pin (1) obtained after cutting presents an irregular surface on which are regularly distributed reliefs which never exceed 10 to 20 micrometers but whose presence is constant, these reliefs being due, at the level of the truncated parts (5, 7) of the pin, to the oblique section of the fibers (5) further to machining, and, on the cylindrical parts, by grooves between the fibers (5) remaining very solidly fixed to the resin matrix (4).

6. Coronoradicular reconstitution assembly according to any one of the preceding Claims, characterized in that the coronary reconstitution material (3) is a self-polymerizing polyurethane-acrylic resin laden with sized short glass fibers.

7. Coronoradicular reconstitution assembly according to Claim 6, characterized in that the glass fibers have a length of 200 micrometers and a diameter of 9 micrometers, about, they are present at a rate of 70 to 80% in volume and are distributed in random manner in the mass of the coronary reconstruction material.

8. Process for manufacturing a root pin for dental prosthesis made from composite material for a coronoradicular reconstitution assembly according to any one of Claims 1 to 7, characterized in that a resin (4) is extruded around a tuft of continuous fibers (5) in equal tension, possibly prestressed in traction, and, after hardening of the resin, the rod of composite material is cut out and the pin is machined mechanically.

## Patentansprüche

1. Vorrichtung zum coronoradikulären Wiederaufbau mit einem physiologischen Zahnankerstift (1) aus Composite-Material und einem Material zum Wiederaufbau der Corona (3) zum Einbringen zwischen dem Stift (1) und den Wänden des gesunden Zahnbeins (2a), wobei der Stift (1) hochfeste Fasern (5) aufweist, die in einer Harzmasse (4) eingebettet sind, welche die Matrix bildet, wobei die Fasern (5) untereinander deutlich parallel und fortlaufend ein Ende am anderen angeordnet sind, dadurch gekennzeichnet, daß sich die Fasern (5) in axialer Richtung des Stiftes (1) erstrecken und gleichgespannt sind in der Weise, daß der erhaltene Stift (1) mechanische Eigenschaften aufweist, die denjenigen des Zahnbeins ähneln, was den transversalen Elastizitätsmodul anbelangt, und bessere als die des Zahnbeins, was den Widerstand gegen Kompression oder Zug in Längsrichtung und den Widerstand gegen Abscheren betrifft.

2. Vorrichtung zur coronoradikulären Wiederherstellung nach Anspruch 1, dadurch gekennzeichnet, daß sie aus Kohlenstoff-Fasern (5) besteht, die in einen Epoxy-Harz-Matrix (4) eingebettet sind.

3. Vorrichtung zur coronoradikulären Wiederherstellung nach einem der vorangehenden Ansprüche, dadurch gekennzeichnet, daß die Fasern (5) einer gleichen Zug-Vorspannung unterworfen werden.

4. Vorrichtung zur coronoradikulären Wiederherstellung nach einem der vorangehenden Ansprüche, dadurch gekennzeichnet, daß die Fasern (5) 40 bis 80 Volumen-% des Gesamtvolumens des Stiftes (1) ausmachen.

5. Vorrichtung zur coronoradikulären Wiederherstellung nach einem der vorangehenden Ansprüche, in der der Stift aus einem Rotationsstück besteht, das aus zwei zylindrisch-konischen koaxialen Teilen, wobei das eine in Vorlängerung des anderen angeordnet ist, gebildet wird, das heißt aus einem oberen zylindrisch-konischen Toll mit großem Durchmesser und einem unteren zylindro-konischen Teil mit kleinerem Durchmesser, dadurch gekennzeichnet, daß der nach der Herstellung erhaltene Stift eine unregelmäßige Oberfläche aufweist, auf welcher sich in regelmäßiger Verteilung Unebenheiten befinden, die 10 bis 20 Mikrometer nicht überschreiten, deren Auftreten jedoch konstant ist, wobei diese Unebenheiten auf dem Niveau der kegekstumpfförmigen Teile (5,7) des Stifts auf den schrägen Abschnitt der Fasern (5) infolge der Bearbeitung zurückzuführen sind, und auf den zylindrischen Teilen, durch Rillen zwischen den Fasern (5), die sehr fest an die Harz-Matrix (4) gebunden bleiben.

6. Vorrichtung zur coronoradikulären Wiederherstellung nach einem der vorangehenden Ansprüche, dadurch gekennzeichnet, daß das Matrial zur Wiederherstellung der Corona (3) ein selbstpolymerisierendes Polyurethan-Acryl-Harz mit einer Verstärkung aus kurzen, eingestreuten Glasfasern ist.

7. Vorrichtung zur coronoradikulären Wiederherstellung nach Anspruch 6, dadurch gekennzeichnet, daß die Glasfasern eine Länge von etwa 200 Mikrometern und einen Durchmesser von etwa 9 Mikrometern aufweisen, etwa 70 bis 80 Volumen-% in der Masse des Corona-Wiederherstellungsmaterials ausmachen und in einer Zufalls-Verteilung darin verteilt sind.

8. Verfahren zur Herstellung eines physiologischen Zahnanker-Stiftes aus Composite-Material für eine Vorrichtung zur coronoradikulären Wiederherstellung nach einem der Ansprüche 1 bis 7, dadurch gekennzeichnet, daß man ein Harz (4) um einen Strang von kontinuierlichen, gleichgespannten und gegebenenfalls zugvorgespannten Fasern (5) extrudiert, nach Erhärtung des Harzes den Strang aus Composite-Material schneidet und den Stift mechanisch bearbeitet.
